# EUROPEAN PATENT APPLICATION

(11) **EP 1 630 170 A1**
(43) Date of publication of application: **01.03.2006**
(21) Application number: 04090326.2
(22) Date of filing: 24.08.2004
(51) Int. Cl.: C07K 7/00, C12N 15/00, C07K 2/00

(54) **A method of generating a crystal of a molecule-of-interest and a kit therefor**

(71) Applicant: Freie Universität Berlin, 14195 Berlin (DE)
(72) Inventor: Vallazza, Marco, Dr., 12437 Berlin (DE); Erdmann, Volker, Prof. Dr., 14197 Berlin (DE)
(74) Representative: Ziebig, Marlene

(57) **Abstract**

The invention relates to a method of generating a crystal containing a molecule-of-interest comprising: (a) contacting said molecule-of-interest with a crystallization carrier, the crystallization carrier being a well-crystallizing molecule structurally different from the molecule-of-interest and not being capable of homomultimerizing, using a heterologous intermolecular linker comprising a carrier-binding site and a molecule-of-interest-binding site, the linker being capable of interlinking crystallization carrier and molecule-of-interest or a recognition signal thereof, thereby forming a crystallizable complex, whereby linker and molecule-of-interest are non-covalently interlinked, and crystallization carrier and molecule-of-interest are exclusively contacted by the linker; and (b) subjecting said complex to crystallization-inducing conditions which are derived from the crystallization conditions of the crystallization carrier and/or a complex consisting of crystallization carrier and linker, thereby generating a crystal containing the molecule-of-interest.

## Description

The present invention relates to a method of generating a crystal containing a molecule-of-interest, a suited polypeptide linker and a kit therefor, and a method of determining the structure of said molecule-of-interest.

Progress in molecular biology and its application to human health are crucially dependent on atomic-resolution structural knowledge of proteins, nucleic acids and other biological macromolecules. Beyond the transforming impact that three-dimensional structures have on the fundamental research in life sciences, the macromolecular structure has proven to be of formidable value in the applied biotechnology. Here, it provides the essential knowledge required to apply the strategy of structure-based drug design to the creation and discovery of novel drugs and pharmaceutical products and serves as the basis for powerful approaches to identify lead compounds to treat numerous human ailments, veterinary problems or crop diseases in agriculture. Thus, the structure determination supplies the analytical function, whereas recombinant DNA techniques are the essential synthetic tool that permits the modification of biological macromolecules. Intelligent and purposeful changes are possible by means of this structural guide, in place of random substitutions. The direct visualization of structural alterations that are introduced by mutations offers new directions for chemical and physical enhancements.
Redundancies in structural elements and motifs emerging from the expanding collection of structurally known macromolecules, particularly proteins, suggest that the number of architectures and substructures naturally occurring is finite and manageable. Once, most of the folds which are utilized by nature are characterized, the knowledge will provide predictive insights into the structure and function of unknown proteins primarily based on their amino acid sequence. The sequences of many proteins from a wide range of organisms are currently defined through massive sequencing efforts, like the human genome project. The extension of such projects to the three-dimensional structural level of the proteome is the next logical step running under the term of structural genomics.

A number of techniques are employed for determining biological structures including X-ray crystallography, nuclear magnetic resonance (NMR) spectroscopy and mass spectrometry. Although the NMR spectroscopy provides a powerful tool for structural investigations in solutions, the method is restricted by a molecular size smaller than 25 kDa and the necessity of specifically incorporating nucleotides which are labeled with isotopes. For example, high-resolution NMR studies of large macromolecules require the accumulation of ¹⁵N- and ¹³C-isotopes therein. At present and in the near future, the only technique that can yield atomic-resolution structural images of biological macromolecules is the X-ray structure analysis as applied to a single crystal. The application of that X-ray crystallography is absolutely dependent on crystals of the macromolecule. However, it is not solely crystals which are required, but such of sufficient size and quality to permit the collection of precise diffraction data to resolutions that allow biochemical insights. The quality of the final structure correlates directly with the perfection, size and physical properties of the crystalline specimen. Hence, the crystal becomes the keystone element of the entire process and the ultimate determinant of its success.

This primary obstacle in structure determination is due to the necessarily empirical nature of the "trial and error" method employed. Macromolecular crystallization is a matter of searching the ranges of individual parameters that impact upon crystal growth by performing countless crystallization trials. The fundamental question of why particular solution conditions finally produce crystals of a specific macromolecule remains unanswered yet. Macromolecules are extremely complex physical-chemical systems which properties vary as a function of many environmental influences such as temperature, pH, ionic strength, contaminants, solvent composition etc. Macromolecules are structurally dynamic, micro-heterogeneous, aggregating systems, and they change the conformation in the presence of ligands. Crystallization conditions have to be carefully fine-tuned to induce the proper molecular conformation and packing orientation of each molecule. Such conditions are difficult to obtain since small variations in the physical-chemical parameters strongly influence the crystallization process in a way that is unique for each biological macromolecule due to the number and diversity of chemical groups and possible configurations thereof involved in the formation of intermolecular contacts. The majority of structure-genomics programs have obtained diffraction-quality crystals for approximately 10 % of the expressed proteins.

Besides proteins, ribonucleic acids are the major class of biological macromolecules interesting to be crystallized. The diverse functions of ribonucleic acids as information carrier (mRNA), adapter (tRNA), or scaffold and catalyst (rRNA) reflect the crucial importance of RNA within the living cell and its significance as essential for life itself. A number of novel properties of RNA molecules make them useful in new applications in biotechnology and molecular medicine. Three examples are noted here. Firstly, the discovery of self-cleaving RNA and natural ribozymes and the development of new catalytic RNA molecules not only support the hypothesis of an early RNA world, but also open up new opportunities for novel clinical applications. Secondly, RNA molecules have also been found to express high affinity for a wide variety of target molecules. Called aptamers, these RNA ligands can be produced by an efficient *in-vitro* selection procedure. Because of their high affinity for a broad spectrum of structural targets, such RNA molecules have properties comparable to antibodies. L-RNA versions of these molecules, called Spiegelmers, are especially long-lived as they are essentially impervious to natural degradation processes. Thirdly, a number of small RNA molecules have been found to interfere with and regulate gene expression. The action of RNA interference on dsRNA to trigger sequence-specific gene silencing is likely to find future application in novel therapeutics against a variety of diseases. These and other structural and functional properties of RNA are being employed in the rapidly evolving area of RNA technology and, as a consequence, have intensified and underscored a great need for more structural understanding of folding motifs in order to design functional RNA molecules.

RNA is even less inclined to crystallize compared to proteins. So far, less than 2 % of known macromolecular structures belong to ribonucleic acids. The simultaneous appearance of multiple RNA conformers caused by fundamental folding problems, the intrinsic flexibility of RNA molecules and the undifferentiated molecular surface are reasons for this reluctant crystallization behavior. Displaying just four side chains instead of twenty, RNA has a paucity of primary structure diversity compared with proteins. Furthermore, the four RNA side chains are more similar to each other than the protein side chains are. The RNA side chains only occur as purine and pyrimidine and each is a planar group decorated with hydrogen bond donors and acceptors, whereas the protein side chains comprise hydrophobic, hydrophilic and charged groups of varying sizes and shapes. The dearth of primary structure diversity of a RNA polymer relative to a protein polymer renders it more difficult for the RNA to specify a unique tertiary structure that is thermodynamically strongly favored over competing structures. Next, the high thermodynamic stability of RNA duplexes results in a tendency to fold into and become kinetically trapped in alternative conformations. Incorrect RNA conformers can be further stabilized by random tertiary interactions with 2'-hydroxyls, phosphoryl groups and metal ions, and by the formation of non-canonical base-base interactions. Additionally, due to sequestering the base-pairing faces of residues in the interior of duplexed regions, RNA has a molecular surface dominated by an essentially uniform array of negative charges of the phosphate backbone hampering intermolecular crystal contacts.

Generally, there are two approaches to exert an influence on the crystallization process: the variation of the external crystallization environment and the alteration of the internal crystallization target. The first one comprises sparse matrix screens tailored for several types of biological macromolecules, like proteins, membrane proteins, nucleic acids, particularly RNA, *etc.* They are commercially available containing either empirically proven solutions or a set of new combinations of favorite components selected from the groups of precipitants, ions, buffers, polyamine *etc.* Due to the huge number of factors affecting crystal growth, sparse matrix screens are never systematically composed as the name suggests. Therefore, the use of a neutral net to predict crystallization conditions via a small sample of screening results has been previously attempted (DeLucas *et al.* (2003) *J. Struct. Biol.* 142, 188-206). More commonly, the high-throughput screening (HTS) on an automated basis and in the submicroliter scale is currently applied to overcome the incompleteness of conditions tested, along with maximizing the crystallization probability of a certain target. Nowadays, HTS represents a component of fully integrated protein crystallization platforms involving HT construct design and screening, HT expression and purification as well as HT crystallization and imaging. For example, over a period of 18 months, Syrrx, a structure-based drug discovery company, has executed several million crystallization and imaging trials on over 400 unique drug-discovery targets. Finally, the structures of 73 macromolecules have been determined and 28 other proteins have yet to be solved. Although, HTS enables increased crystal hits, the success rate is far away from 100 %. It is becoming clear that the effectiveness of crystallization experiments is not directly proportional to the number of conditions tested. Instead, the sample amount and the technical efforts are tremendous.
Since the majority of crystallization targets is shown as not crystallizable readily, target improvements are necessary. Several methods have been proposed to obtain crystallizable molecules. The conformational changes induced upon ligand binding may be favorable to the crystallization process by exposing new crystal contacts or by stabilizing the crystallization target. Complex formation of proteins and subsequent co-crystallization screens can be performed with co-factors, inhibitors or even antibody fragments. RNA molecules can be co-crystallized with natural binding partners, comprising proteins, substrates and analogues, or high-affinity targets. However, complexation depends on the availability and type of such binding partners and provides no guarantee of choosing one which supports crystal growth. If protein partners do not exist or are experimentally intractable, a protein-binding motif including the stem loop 2 of U1 RNA can be introduced into a RNA molecule. The RNA can subsequently be co-crystallized with the appropriate RNA-binding protein, the RNA-binding domain of the U1A protein. Because of the small size of U1A, this protein is considered as surface alteration element (Ferré-D'Amaré & Doudna (2000) *J. Mol. Biol.* 295, 541-556). The extension of RNA sequence may also interfere with the appropriate RNA folding into an active conformation.
As denoted, molecular biology has become a powerful tool to engineer biological macromolecules in order to improve their crystallization features. Such modifications could be site-directed point mutations, preferably to engineer surface properties that favor the formation of intermolecular crystal contacts. Deleting or removing the flexible regions at the termini or within a protein can help in the crystallization by minimizing any interfering effects from the microheterogeneity. Unfortunately, in both cases a basic idea of the macromolecular structure is required in advance.
A strategy for crystallizing RNA motifs which is least disruptive to the overall structure includes helix engineering comprising the variation of helix length and the number and type of overhanging nucleotides. Other areas to be addressed are the number and type of nucleotides in loops, bulges and stems. Replacement of natural sequences with RNA tetraloops is commonly used, because these motifs provide a compact structure and are known to mediate RNA packing. Addition of heavy atoms to the major groove may open the groove making further contacts with other RNA molecules possible (Anderson *et al.* (1996) *J. Mol. Biol.* 259, 696-703). Nevertheless, the success of all approaches is rather randomly.
In consequence, a general module for RNA crystallization consisting of a GAAA tetraloop/ tetraloop receptor has been designed for promoting intermolecular contacts (Ferré-D'Amaré *et al*. (1998) *J. Mol. Biol*. 279, 621-631). The necessity of incorporating it into the crystallization target could destroy the functional target conformation and the number of possible arrangements within the target enhances the cloning and screening efforts, therefore limiting the application.
Finally, fusion constructs have been designed. Their crystallization is driven by a crystallization tag, but there are very few examples of fusion proteins being crystallized (Dale *et al.* (2003) *J. Struct. Biol.* 142, 88-97). The choice of the linker is critical and has to be taken individually for each protein. The addition of intramolecular linker segments causes an inherent flexibility of the resulting subunits. The often unknown structure of the linker prevents the prediction of folding with regard to the desired appearance of independent subunits. The latter is further complicated by the integral coupling of the units. Last, the accessibility of one terminus of the crystallization target is an absolute requirement for linker coupling. At present, neither suited methods nor linkers are available to contact RNA to a crystallization tag.
A specific embodiment of fusion constructs discloses the patent application WO 03/016330 by using molecular linkers to interlink at least two molecules-of-interest, preferably membrane proteins, for geometry-driven crystallization. However, it seems practically difficult to achieve the desired uniform loading or saturation of linker binding sites with the molecule-of-interest resulting in the defined geometry.

All prior art approaches have failed to provide an adequate solution for efficiently generating X-ray diffraction grade crystals of macromolecules. Therefore, the technical problem forming the basis of the present invention is to provide a method and tools for generating crystals of macromolecules which avoid the above-mentioned limitations of material- and time-consuming screening for suitable crystallization conditions and suitable crystallizing mutants of a molecule-of-interest. It is a further object of the present invention to provide a method and tools which either generate crystals of a molecule-of-interest better suited for X-ray structure analysis or give crystal hits of a molecule-of interest not able to be crystallized another way.

The present invention solves this problem by providing a method of generating a crystal containing a molecule-of-interest, comprising:
a) contacting said molecule-of-interest with a crystallization carrier, the crystallization carrier being a well-crystallizing molecule structurally different from the molecule-of-interest and not being capable of homomultimerizing, using a heterologous intermolecular linker comprising a carrier-binding site and a molecule-of-interest-binding site, the linker being capable of interlinking crystallization carrier and molecule-of-interest or a recognition signal of the molecule-of-interest, thereby forming a crystallizable complex, whereby linker and molecule-of-interest are non-covalently interlinked, and crystallization carrier and molecule-of-interest are exclusively contacted by the linker; and
b) subjecting said complex to crystallization-inducing conditions which are derived from the crystallization conditions of the crystallization carrier and/or a complex consisting of crystallization carrier and linker, thereby generating a crystal containing the molecule-of-interest.

The method according to the present invention is based on the crystallization of molecules-of-interest by crystallizing them in complex with a crystallization carrier susceptible to provide an ordered matrix facilitating the formation of the basic unit of a crystal lattice. An easily crystallizable molecule with a well-characterized three-dimensional structure is chosen as carrier which size is preferably larger than that of the molecule-of-interest. Crystallization of the molecule-of-interest is driven by the crystallization of the crystallization carrier. In particular, carrier-driven crystallization facilitates the crystallization of molecules-of-interest that are either difficult to crystallize or do not crystallize at all.
In contrast to the prior art which describes the creation of fusion constructs using an intramolecular linker to interlink the molecule-of-interest with a crystallization tag by covalent interlinkage at the molecule-of-interest-binding site or at both linker sites, the method of the present invention advantageously applies intermolecular linkers to interlink the molecule-of-interest with a crystallization carrier. The "intermolecular linker" of the present invention refers to a structural element which is independent from the molecule-of-interest due to a non-covalently interlinkage of the molecule-of-interest with the molecule-of-interest-binding site of said linker, or additionally independent from a crystallization carrier by contacting the molecule-of-interest with the crystallization carrier by non-covalent interlinkages at both linker sites, the molecule-of-interest-binding site and the carrier-binding site, thereby forming a ternary and crystallizable complex. "Non-covalent" interlinkage comprises hydrophobic/hydrophilic interactions, van der Waals forces, ion pairs, ligand-receptor interactions, interactions between epitope and antibody binding site, nucleotide base pairing, and the like.
The application of intermolecular linkers enables the correct, native folding of carrier and molecule-of-interest independently from each other. Furthermore, the exclusive indirect contact of carrier and molecule-of-interest by the linker results in a minimum of or none structural interference. Due to the separated spatial folding of carrier and molecule-of-interest, each approved pair of crystallization carrier and linker is generally applicable. The time-consuming individual selection of a carrier/linker pair suitable for forming a crystallizable complex with a specific molecule-of-interest becomes superfluous. Simultaneously, the efforts of preparing components for the crystallizable complex are reduced.

Subjecting said complex of molecule-of-interest and crystallization carrier to crystallization-inducing conditions, which are derived from the crystallization conditions for the crystallization of the crystallization carrier and/or from the crystallization conditions for the crystallization of a complex containing crystallization carrier and linker, is of advantage to diminish the screening efforts. The "crystallization-inducing condition" comprises the hardware, crystallization method, physical parameters and the crystallization solution. The hardware mainly depends on the crystallization method, for example 24 well plates (Linbro®), CrystalClear stripes and HDPCG assembly for vapor diffusion, thereby determining the surface of crystallization vessels, the distance between reservoir solution and sample etc. Crystallization methods are vapor diffusion, batch technique, dialysis, free interface diffusion etc. Currently, the mostly used crystallization method is vapor diffusion, preferably the hanging drop crystallization. Physical parameters involve temperature, volume of crystallization drop, gravity, convection and sedimentation, vibrations and sound, pressure, electric and magnetic fields, viscosity etc. With the exception of temperature and drop volume, they are fixed by the terrestrial environment and rather seldom changed, e.g. in microgravity experiments. Drop size exerts an influence on the kinetics. Temperature acts via dielectric constant, solution energy and entropy on solubility and diffusion. Usually, crystals are grown at 4°C or room temperature. In the meaning of the invention, the crystallization method, corresponding hardware, drop size and temperature should be selected in accordance to those of the carrier or a complex of carrier and linker, or optionally follow the cited statistics. The crucial factor for crystallization success is represented by the crystallization solution comprising diverse influencing variables, such as buffer and pH, ionic strength, concentration and nature of precipitant, additives, reducing or oxidizing agents, metal and other specific ions, surfactants or detergents etc. For example, crystal packing caused by electrostatic interactions is affected by the charge of a molecule-of-interest which could be manipulated via the pH value of the buffer. Although, the majority of known protein crystals appeared around a neutral pH, differences less than 0.5 pH units are critical. Among the precipitants, organic solvents, salts and polymers are most commonly used. The increase of electrostatic attraction by diminishing the electric constant in presence of organic solvents (e.g. ethanol, iso-propanol, 1,6-hexanediol, MPD), the removal of water by high ion strength (e.g. salting out with ammonium sulfate), the combination of both by polymers (PEG), and other active principles require individual testing for a molecule-of-interest. In the meaning of the invention, a clue is provided by the crystallization solution of the carrier and/or a complex of carrier and linker.
It was found that the carrier-driven crystallization of the complex largely follows the crystallization of the carrier as single molecule. The initial crystallization conditions comprise the optimum conditions for the carrier crystallization considering hardware, method, physical factors and composition of the crystallization solution. The physical parameters and the solution components are rather systematically changed to account for the influence of the molecule-of-interest during complex crystallization, therefore giving hits in the initial crystallization trials with the utmost probability. Savings in time for screening and imaging, expensive sample material and crystallization solutions are the deserving result.

According to the invention the molecule-of-interest is a biological macromolecule, preferably a protein or a nucleic acid. Proteins and nucleic acids are biological macromolecules often defying crystallization which represents the basic requirement for determining the structure of such a molecule by X-ray structure analysis. The urgent need for structural information to get detailed insights into the structure/function relationship has already been discussed. Therefore, it is of particular advantage to get such molecules of high biological impact, but intractable crystallization behavior, crystallized.

In another preferred embodiment of the present invention, a ribonucleic acid is used as molecule-of-interest.
One fast expanding area in biotechnology is that of RNA technology contracting the inherent structural and functional potentials and features of RNA molecules for the development of ribozymes, aptamers, Spiegelmers or an *in-vitro* system for the synthesis of proteins. However, the structure determination of RNA molecules turns out to be extremely difficult by several reasons. The simultaneous appearance of multiple RNA conformers and an intrinsic flexibility of RNA molecules are mainly observed in the isolated state. Advantageously, the binding via induced fit supports the functional conformation and often enhances stability as performed by using the heterologous intermolecular linker of the invention. Eventually, by interlinking said RNA with the linker of the invention, the former undifferentiated molecular surface is altered and by contacting said RNA with the crystallization carrier of the invention, further new crystal contacts are promoted.

According to the present invention, the crystallization carrier is a biological macromolecule, preferably a protein or a nucleic acid, and more preferred a protein. Proteins are biological macromolecules which can be expressed at high levels and which are easily purified by known standard techniques. Referring to the **R**esearch **C**ollaboratory for **S**tructural **B**ioinformatics (RCSB) **P**rotein **D**ata **B**ank (PDB), there are numerous examples of well-crystallizing and structurally characterized proteins. With regard to the molecule-of-interest a handling of the carrier under adequate conditions is advantageously desired while crystallizing. Furthermore, a potential high molecular weight of the macromolecules-of-interest requires an adequate or preferably higher molecular weight of the crystallization carrier. The latter could be obtained by fusion to a second carrier molecule.

Preferred proteins used as crystallization carriers are calmodulin, S-protein or monomeric streptavidin.

In recent years, several epitope peptides and proteins have been developed to purify over-expressed recombinant proteins. Among others, they share the features of a minimal effect on tertiary structure and biological activity as well as the applicability to a number of different proteins. Unfortunately, there are only a few examples in which the matrix represents a crystallized and structurally characterized protein which is bound with nanomolar affinity by a peptide tag (Terpe, K. (2003) *Appl. Microbiol. Biotechnol.* 60, 523-533). The latter is regarded as essential for the present invention in order to form a stable crystallizable complex even under crystallization conditions which are not optimal for the affinity tag/matrix recognition or the linker/crystallization carrier interlinkage, respectively, as expressed in terms of the invention.

Preferably, calmodulin or S-protein is used as crystallization carrier. Calmodulin consists of about 148 amino acid residues and is strongly bound in the presence of 0.2 mM CaCl₂ by the calmodulin-binding peptide having 26 residues. The crystallization conditions are known and the structure was determined (Meador *et al.* (1992) *Science* 257, 1251-1255). In particular, this crystallization carrier is well-suited for the crystallization of prokaryotic proteins as molecules-of-interest because they do not interact with calmodulin in a calcium-dependent manner. As explained above, any cooperative interaction has to be excluded to avoid a structural impact.
The S-protein consisting of 103 amino acid residues and its cognate S-tag having 15 amino acids are both derived from RNase A. The S-protein/S-tag complex has a dissociation constant of about 100 nM which depends on pH, temperature and ionic strength. The tag is composed of four cationic, three anionic, three uncharged polar and five non-polar residues. This composition makes it soluble. The crystallization conditions are known and the structure was determined (Kim *et al*. (1992) *Biochemistry* 31, 12304-12314). Since S-tag binding is accompanied by the reconstitution of ribonucleolytic activity, the system is applicable to such a molecule-of-interest which does not represent a substrate for a nucleolytic cleavage reaction. Advantageously, the system is suited to crystallize a molecule-of-interest comprising proteins from bacteria, mammalian cells and baculovirus-infected insect cells.

Streptavidin from *Streptomyces avidinii* is a homotetrameric protein with a biotin binding site in each subunit of 159 amino acids. It binds D-biotin with a remarkably high affinity of 10⁻¹⁵ M. In addition, a number of different streptavidin-binding peptides in the length of 8 to 38 amino acids are known. All of them share the HPQ motif and can be selectively eluted from streptavidin by the addition of biotin. They are discussed in detail below - just as the novel Nano-tag. Streptavidin can be easily crystallized (Schmidt *et al*. 1996 *J. Mol. Biol*. 255, 753-766) by ammonium sulfate precipitation at a neutral pH value (well D2). Taking the given reference as starting point, solutions could be derived for the crystallization-inducing screening of the crystallizable complex of the present invention (Table 1). The variation includes an increase of the precipitant concentration (wells E2, F2, A3) which is also tried at lower (wells C1, D1) and higher pH values (wells A4, B4, C4). In addition to the mechanism of salting out, PEG is used as another precipitant which combines impact on the dielectric constant, dehydration and restructuring of the solvent. PEGs of several molecular weights and derivatives thereof are included at different pH values, in particular within a range of ± 0.5 units at pH 7.0. The ionic strength and the supply of counter ions is optionally regulated by adding salts which provide different types of mono- and divalent ions (all other wells). Despite the great binding affinity of biotin, it is proven to be difficult to obtain a complete saturation of binding sites in the homotetrameric structure. A possible reason is a hampered accessibility of subunits by a steric interference of molecules themselves to be bound. The resulting non-equivalence of loading will prevent the regular build-up of the crystal lattice.
To overcome the geometric obstacle, the present invention suggests the use of streptavidin which is not being capable of homomultimerizing as crystallization carrier. The term "homomultimerizing" denotes an association of molecules involving at least two crystallization carriers which are originated from the same species, thereby forming a stable and functional higher order complex. The term "association" means any type of interaction between two molecules in the context of the present invention.
According to the invention it is especially preferred to use monomeric streptavidin generated by mutation as crystallization carrier. Natural tetrameric streptavidin has two subunit interfaces: one is a strong interface between subunits in a tightly associated dimer (monomer-monomer interface), and the other is a weak interface between a pair of such dimers (dimer-dimer interface). Several attempts are performed to engineer subunit association resulting in reduced biotin-binding affinities of the dimeric and monomeric streptavidin. For example, this phenomenon is caused by the fact that the side chain of tryptophan in position 120 makes intersubunit contacts to biotin bound by an adjacent subunit through the dimer-dimer interface in natural streptavidin. However, the affinity to biotin is still in the nanomolar range required for the present invention. It is not excluded that there are other positions for mutating streptavidin in order to produce monomeric streptavidin while maintaining the original affinity or at least a nanomolar affinity. The homology between native core streptavidin and mutated streptavidin should amount to at least 70 %, whereby the number of mutations is inevitably limited by the requirement of the aforementioned binding affinity. Possible mutations include deletion, insertion, substitution, modification and addition of at least one amino acid, or the fusion with another protein. Furthermore, the deep permeation of the fM-Nano-tag and derivatives thereof into the biotin-binding pocket of streptavidin obviously maintains the strong interaction in the monomeric state. fM is the abbreviation for formyl-methionine.
It might also be possible to maintain native monomeric streptavidin by providing a chemical environment which prevents the association to the tetrameric conformation.

In a most preferred embodiment of the invention, mutated streptavidin harboring at least the amino acid substitution of aspartate to alanine in position 128 is used as crystallization carrier. The impact of individual residues on the overall structure of streptavidin is reflected by the formation of monomeric streptavidin to different extents. Among several targeted residues, Asp-128 has the most dramatic effect on subunit association, along with the best remaining dissociation constant. Its substitution to alanine results in 80 to 88 % of monomeric streptavidin which can be easily separated from the tetrameric form by HPLC (Qureshi *et al*. (2001) *JBC* 49, 46422-46428). However, other kinds of mutation in position 128 of streptavidin, such as deletion, modification of the amino acid, e.g. alkylation, arylation and acetylation, or substitution by another amino acid than alanine are also encompassed by the invention. The aforementioned mutations may also concern other framework structure-maintaining residues of streptavidin, preferably those which are involved in tight interactions of the monomer-monomer interface observed in tetrameric streptavidin or a series of hydrogen-bonding interactions, more preferably the eight residues Asn-23, Ser-27, Tyr-43, Ser-45, Asn-49, Ser-88, Thr-90 and Asp-128.

According to the method claimed by the present invention, the crystallization of a molecule-of-interest is effected by contacting said molecule-of-interest with a crystallization carrier using a heterologous intermolecular linker. In the meaning of the invention, a "heterologous linker" is a linker wherein at least one constituent, such as the carrier-binding site and/or the molecule-of-interest-binding site, is derived from a source which differs from the source of the other components of said construct and/or said complexes. For example, the molecule-of-interest as part of the complex is a RNA and the linker is a polypeptide, the carrier-binding site of which could be a synthetic fM-Nano-tag and the molecule-of-interest-binding site could be a RNA-recognizing polypeptide, such as Hsp15, (AAKK)₄, PUM-HD, PABP *etc*. As already defined, an "intermolecular linker" refers to a non-covalently interlinkage at least of the molecule-of-interest with the molecule-of-interest-binding site of said linker.
A linker of the present invention usually comprises two subunits termed sites. In the meaning of the invention, the term "site" relates to a certain part of a linker which is able to interlink said linker with the crystallization carrier or the molecule-of-interest or parts thereof. Site and carrier or molecule-of-interest, respectively, could belong to the same molecular species, e.g. being polypeptides, or different ones, e.g. being a polypeptide linker and a RNA as molecule-of-interest. Such a site could either consist of a single molecular building block, like a nucleotide or an amino acid, which is preferably covalently interlinked with the crystallization carrier, or comprise several molecules, like a polynucleotide or an amino acid sequence, which is preferably non-covalently interlinked with carrier or molecule-of-interest.
Carrier-binding site and molecule-of-interest-binding site interlink crystallization carrier and molecule-of-interest which are different. Furthermore, both linker site are of different origin. However, a linker site and its cognate carrier or molecule-of-interest, respectively, can also be originated from the same source (refer to S-protein/S-tag). The linker could be designed to contain any desired binding domains according to the carrier and the molecule-of-interest to be contacted. Preferably, a linker is used which carrier-binding site is non-covalently and specifically interlinked with the crystallization carrier. The folding of carrier and molecule-of-interest into native conformations within the crystallizable complex is of great advantage for functionality and ensured by non-covalent interlinkage. The sequence-specific attachment mostly provides a binding affinity which prevents the development of heterogeneous solutions from any complex dissociation. "Heterogeneous" refers to a solution which is composed of at least two components. The solution can be composed of a molecule and fragments thereof and/or molecules of different origin.

Crystallization carrier and carrier-binding site could be covalently and specifically interlinked as well. For example, a fusion protein containing aforementioned parts is expressed.
Finally, a non-specific interlinkage to the crystallization carrier might be possible. For example, certain peptides, polypeptides or aptamers are able to interact with molecules-of-interest regardless their primary structure.

In another preferred embodiment of the invention, a linker is used which carrier-binding site and molecule-of-interest-binding site are covalently bound.
This kind of linker is highly stable, rigid and can be advantageously used to generate crystallizable complexes without conformational disorder. Usually, covalent linkage is the tightest attachment of two compounds and therefore it could restrain a flexible linker structure. A minimum in linker size correlates with a maximum in tightness of packing of the molecular complex. The linker production and purification is also facilitated and less expensive. In case, that carrier-binding site and molecule-of-interest-binding site consist of the same type of molecule, such as a peptide, the linker can be easily synthesized ready-for-use.

In another preferred embodiment of the present invention, a linker is used which contains a spacer molecule between the carrier-binding site and the molecule-of-interest-binding site.
The spacer molecule could be necessary to enable the correct and independent structural arrangement of the binding sites as well as to guarantee the accessibility of both sites. Blocking, structural interference or impact by cooperative binding of carrier and molecule-of-interest are to be avoided thereby. The spacer is preferably composed of the same molecule species as the carrier-binding site and/or the molecule-of-interest-binding site are, e.g. being a single amino acid or a peptide, or, a single nucleotide or polynucleotide sequence. In addition, a molecule species differing from the other constituents of the linker is imaginable as spacer.

In another preferred embodiment of the invention, a linker is used which carrier-binding site is a peptide.
Peptides are especially preferred by several reasons. They can be quickly synthesized chemically, *in-vivo* or *in-vitro* and modified, for example to alter the binding affinity. Modifications may comprise mutations of amino acids, chemical treatments or the coupling of functional groups, such as for fluorescence labeling. Advantageously, a peptide carrier-binding site can be easily connected with a molecule-of-interest-binding site which is a peptide as well by synthesizing the complete linker at once. The high stability is achieved by the rigid peptide bond.
Either, the carrier-binding site, along with the crystallization carrier, could be obtained by splitting a well-crystallizing protein as performed for the S-protein/S-tag. Or, in principle, a peptide ligand could be selected against any well-crystallizing molecule, preferably a protein which is used as crystallization carrier in the meaning of the present invention. A number of different methods have been developed to screen a peptide or protein library for specific binders. The majority of these methods, namely phage display, cell surface display, plasmid display and the yeast two-hybrid system are so-called *in-vivo* methods because living cells are involved in the process of library generation or screening. To eliminate the *in-vivo* limitation of library complexity caused by the transformation efficiency, the cell-free translation is introduced to *in-vitro* display technologies. The ribosome display achieves the link between genotype and phenotype by expressing proteins from mRNA lacking a stop codon and using the ribosomal complex, consisting of protein, ribosome and mRNA (PRM-complex) for selection. In this way, the streptavidin-binding peptide termed Nano-tag has been generated, for instance, which is described below.
However, it is also possible to redevelop complete systems of crystallization carrier and carrier-binding site of the linker. For this purpose, new molecules from the lab routine will be characterized, crystallized and structurally analyzed, preferably proteins. Subsequently, a ligand has to be generated, preferably a peptide ligand by means of one of the aforementioned methods, or a RNA ligand called aptamer. The latter is identified by an *in-vitro* selection procedure ― the SELEX process (systematic evolution of ligands by exponential enrichment). Since RNA is very susceptible to nucleolytic degradation in biological solutions, aptamers are chemically modified using phosphorothioates, locked nucleic acids or Spiegelmers. Because of their high affinity for a broad spectrum of structural targets, aptamers act very similar to antibodies.
In a preferred embodiment of the present invention, monomeric streptavidin is used as crystallization carrier and a linker is used which carrier-binding site is a streptavidin-binding peptide.

The streptavidin/biotin system represents one of the most tightly bound protein/ligand pairs in nature. However, its practical use is limited by the essential coupling of biotin to any molecule, preferably a peptide, a polypeptide or a protein. Peptides are commonly labeled with biotin during chemical synthesis. The N-terminal attachment is performed via a spacer, such as 6-aminohexanoic acid, which could make the linker too flexible with regard to the present invention. Polypeptides and proteins are biotinylated by standard bioconjugate techniques using biotin-containing chemicals, but is runs randomly. Alternative techniques have been developed for site-specific labeling requiring peptide tags in which the covalent attachment of biotin to a specific lysine residue is catalyzed by biotin ligase.
Preferably, streptavidin-binding peptides are used as crystallization carrier according to the present invention. The majority of known streptavidin-binding peptides either express a low binding affinity or they are uncomfortable large for practical application. For example, the binding affinity of the *Strep*-tag II to streptavidin is fairly weak (dissociation constant of 72 µM) resulting in the development of a streptavidin mutant named *Strep*Tactin expressing a dissociation constant of about 1 µM to *Strep*-tag II. Nevertheless, the aforementioned peptide can be applied in the method of the present invention, but its binding affinity may cause a heterogeneous solution due to partial complex dissociation which disturbs the crystallization of the remaining complex. The SBP(streptavidin-binding peptide)-tag is another peptide that binds to streptavidin with a dissociation constant of 2.5 nM. Despite the high affinity, the peptide is unwieldily because it is 38 amino acids long and consequently rather big to form a stable linker structure.

More preferably according to the present invention, a linker is used which carrier-binding site is a fM-Nano-tag. The term Nano-tag relates to a novel streptavidin-binding peptide family which was created by the selection of a peptide library genetically fused to the N-terminus of bovine heart fatty acid binding protein. The Nano-tag combines the high affinity of the SBP-tag with the small size of the *Strep*-tag. For example, binding experiments revealed a dissociation constant of 4 nM which was expressed by a 15-mer peptide to streptavidin. As suggested by the nanomolar affinity and in accordance with the length, the peptide was termed Nano-tag₁₅. A shortened variant of this peptide with a slightly decreased affinity toward streptavidin (dissociation constant of 17 nM) was designated Nano-tag₉ (Lamla & Erdmann (2004) *Protein Express. Purif*. 33, 39-47). Structural studies revealed surprisingly the significance of an initial formyl-methionine residue at the N-terminus of the Nano-tag for the high-affinity arrangement of the tag residues within the biotin-binding pocket of streptavidin. Furthermore, the hydrophobic side chain of methionine is strongly bound by a couple of interactions with Trp-79, Trp-92, Trp-108, Leu-110 and Trp-120 (2^{nd} molecule) of streptavidin, whereas the formyl group is involved in hydrogen-bonding to Gln-25, Trp-108, Trp-120 (2^{nd} molecule) and Asp-128 of streptavidin.
Therefore, the present invention also comprises a streptavidin-binding peptide termed fM-Nano-tag with the SEQ ID NOs: 1 to 10 according to the sequence listing which includes formyl-methionine (fM) at the N-terminus, the nucleic acid sequence thereof and a vector including said nucleic acid sequence. The choice of vector in accordance to the expression system, the cloning of the claimed streptavidin-binding into said vector containing at least one restriction site, and the control of expression by a suited promoter and/or operator and terminator is performed by standard techniques known to those skilled in the art. The invention also relates to a method of producing such a fM-Nano-tag and derivatives thereof in a cell-based or cell-free system for protein biosynthesis. The resulting product of translation is contacted with immobilized streptavidin and bound at it. After the separation of the solution containing substances which have not bound to streptavidin, the translation product is eluted. Suited elution agents are biotin or derivatives thereof, for example iminobiotin, desthiobiotin and diaminobiotin. It is obvious that the streptavidin-binding peptide of the invention can be produced by chemical synthesis as well. The invention also relates to the usage of the above described fM-Nano-tags (SEQ ID NOs: 1 to 10) as carrier-binding site of the linker to streptavidin in the described crystallization method of the present invention. Due to the short length and the distinct interaction in the biotin-binding pocket of streptavidin, the structure of the fM-Nano-tag and possible derivatives, such as a methionine-Nano-tag, is considered as highly stable and less flexible, therefore being e.g. a well-suited component of the crystallizable complex of the present invention.
The fM-Nano-tags of the invention can be also used for the purification or labeling of a protein produced in a system for protein biosynthesis. A nucleic acid sequence encoding said streptavidin-binding peptide and a target protein is transcribed and translated. The following procedure of purification has already described in the context of the peptide production. The labeled protein is contacted with a streptavidin-conjugate and bound at it. Preferably, the conjugate contains a reporter molecule, e.g. radioactive isotopes, fluorescent substances or enzymes, for detection and quantitation, e.g. on Western blots or in ELISAs. Furthermore, the streptavidin-binding peptide of the invention is used for immobilization of proteins to protein lattices containing streptavidin, thereby generating biochips. In all applications, the peptide tag is situated either at the N- or the C-terminus of the target protein, preferably at the N-terminus.

According to still further embodiments of the present invention, a linker is used which molecule-of-interest-binding site is either directly interlinked with the molecule-of-interest or a recognition signal thereof. In the meaning of the invention, the "recognition signal" denotes a specific part of the molecule-of-interest which is of low molecular weight, like a peptide or a short ribonucleic acid fragment. It is coupled to the molecule-of-interest either covalently or non-covalently. The signal is recognized by the molecule-of-interest-binding site of the intermolecular linker non-covalently and with high affinity, thereby interlinking the molecule-of-interest with said linker.
Taking the first case of direct interlinkage, the molecule-of-interest is immediately applied as it is without any modifications for attachment. Both, the native folding and the proper interlinkage with the molecule-of-interest-binding site are ensured. Generally, any molecule-of-interest expressing a binding affinity to any other molecule can be directly attached to the linker, preferably those molecules which binding affinity is nanomolar and more preferably even lower. In addition, the molecule-of-interest binding site should be easily bound to the carrier-binding site, preferably both sites are composed of the same type of molecule, more preferably the sites are peptides, polypeptides or nucleic acid fragments. For example, the molecule-of-interest is a functional nucleic acid comprising aptamers, ribozymes, DNA enzymes, small interference RNAs (siRNA) etc. and the molecule-of-interest-binding site is the cognate target, substrate or analogue, protein binding partner or complementary strand etc. Hereinafter, some examples are given:
Aptamers can be composed either of DNA or RNA, preferably RNA since the available 2'-hydroxyl group promotes a couple of intra- and intermolecular contacts, the latter being between molecules of the same sequence, different sequences or between RNA and any other molecule which is not composed of RNA. Aptamers are able to bind proteins (e.g. HIV-1 Rev protein, thrombin, human chorionic gonadotropin and the vascular endothelial growth factor), peptides (e.g. the calcitonin-gene related peptide) and small molecules, such as organic dyes (e.g. anthraquinone-based dyes), nucleotides (e.g. ATP), amino acids (e.g. arginine), vitamins (e.g. B12), alkaloids (e.g. theophylline) *etc.*
Catalytic-active ribonucleic acids called ribozymes are distinguished into natural ones including the self-splicing group I introns (e.g. the *Tetrahymena thermophilia* P4-P6 domain), and group II introns, RNase P for processing tRNA, the ribosome for peptide bond formation, the hammerhead ribozyme, hairpin ribozyme, hepatitis delta virus ribozyme *etc.* and artificial ones like the *in-vitro* selected leadzyme. The four last-mentioned ribozymes bind and cleave RNA substrates depending on the recognition sequence to be created.
DNA enzymes, e.g. the 10-23 DNAzyme, are highly sequence-specific, but generalizable to cleave almost any RNA substrate. For crystallographic studies, the substrate is to be modified by a methyl group at the cleavage position. In accordance with the substrate recognition of ribozymes, the non-covalent linkage is performed via hybridization.
RNA interference is a response to dsRNA which triggers the sequence-specific gene silencing. As integral part of the RNA-induced silencing complex (RISC), siRNAs interact with binding proteins which could be chosen as molecule-of-interest-binding site. Furthermore, the single strands of siRNA may act as molecule-of-interest-binding site or molecule-of-interest, respectively, which are hybridized. This principle of splitting the nucleic acid into a molecule-of-interest-binding site and the molecule-of-interest can be transformed to other nucleic acids as well.

In a further embodiment, a linker is used which molecule-of-interest-binding site interacts directly and sequence-specifically with the molecule-of-interest.
High-affinity binding, preferably in the nanomolar range, is caused by sequence-specific recognition. More preferably, this embodiment involves peptide targets as molecule-of-interest-binding site recognized by aptamers and peptide ligands which bind to the cognate target as molecule-of-interest.

In another embodiment, it is possible to use a linker which molecule-of-interest-binding site interacts directly and sequence-independently with the molecule-of-interest.
The sequence-independent binding enables the binding of an entire class of molecules. Once developed and approved, a complex of crystallization carrier and said linker, preferably a polypeptide, could be universally applied. There are two possible mechanisms of recognition: First, the structural scaffold which all molecules have in common could be recognized. For example, a polypeptide of the invention would target the sugar/phosphate-backbone of RNA, preferably within the major groove. Secondly, the polypeptide is directed to three-dimensional folding motifs which often occurs or which are typical for a certain class of molecules. For example, the Cys₂His₂ zinc finger unit is generally fold into a ββα motif to be addressed by a peptide ligand.

In a further preferred embodiment of the present invention, a linker is used which is a polypeptide or which molecule-of-interest-binding site is a polypeptide or a peptide due to the same general reasons already given for a peptide carrier-binding site.

In another preferred embodiment of the present invention for generating a crystal containing a RNA, a linker is used which molecule-of-interest-binding site is a RNA-backbone recognizing ligand or a fragment thereof.
Generally, such a peptide or polypeptide ligand can be selected against any molecule. Among the different methods which have been developed to screen a peptide or protein library for specific binders are phage display, cell surface display, plasmid display, the yeast two-hybrid system and ribosome display.

The present invention also takes advantage of a previous observation that a cationic peptide modeled after the surface of staphylococcal nuclease can attach oligonucleotides and enhance the hybridization to complementary DNA sequences. More preferably, the short lysine-rich peptide (AAKK)₄ is used as molecule-of-interest-binding site of the linker. This peptide was shown to attach to DNA and RNA while delivering a nucleophile. Presumably, the mechanism of recognition involves electrostatic interactions between the peptide and the repetitive anionic phosphodiester backbone of the nucleic acid, and the pairing of two lysine residues in tandem is well-suited for promoting nucleic acid association. In addition to the importance of a specific combination of amino acids and charge distribution in this system, there are more critical considerations, like the spatial orientation and stereochemistry, for future work aimed at the design of more elaborate peptide ligands (Bergstrom *et al*. (2001) *Chem. & Biol*. 8, 199-205). The use of such peptides as molecule-of-interest-binding site of the present invention will also modify the RNA surface possibly creating novel crystal contacts. That means that they do not only act as molecule-of-interest-binding site, but also as crystallization enhancer. "Crystallization enhancer" denotes the molecule-of-interest-binding site or parts thereof which could alter the crystallization behavior of the molecule-of-interest by its association maybe changing surface contacts in the crystal which induce and/or promote crystal growth, thereby increasing the probability of crystallization for a certain molecule-of-interest.
The enhancer function might be fulfilled by the single peptide as well. Lysine is known as one of the least favored residues in crystal contacts, therefore recommended to be mutated to any other positively charged amino acid, for example to glutamine representing one of the most favored residues found in crystal contacts.

Finally, natural-occurring RNA binders could be used as molecule-of-interest-binding site. The majority of RNAs interacts with polypeptides to perform their tasks in the living cell. RNA binding polypeptides are a diverse group which structures vary widely from each other. They have evolved multiple binding strategies, but there are only a few examples for non-specific interaction, preferably the λ phage cro repressor and more preferably Hsp15 which has already been crystallized, therefore acting as crystallization enhancer similar to (AAKK)₄. Hsp15 is an abundant heat shock polypeptide that binds non-specifically with low micromolar dissociation constants to different kinds of nucleic acids. The primary globular domain of Hsp15 is a 90 residues α+β structure that contains a highly conserved region extending from α1 through β3. This distinct structural motif is defined as the αL motif consisting of 49 amino acids rich in arginines at the proposed RNA-binding surface. The minimal nucleic acid sequence required for strong binding is about six nucleotides, but the precise mechanism is still to be illuminated (Staker *et al*. (2000), *EMBO* 19, 749-757).

Taking up the aforementioned second case, using a linker which molecule-of-interest-binding site is interlinked with the molecule-of-interest via a recognition signal bound to the molecule-of-interest, this strategy will be useful for the interlinkage of biochemically important molecules-of-interest for which binding partners do not exist or are experimentally intractable.

In a further embodiment, a linker is used which molecule-of-interest-binding site interacts sequence-specifically with the recognition signal. Preferably, a RNA molecule will be equipped with a signal sequence which is recognized by a polypeptide. Such a polypeptide also places crystal contacts into the uniform charge distribution on the RNA surface which may additionally enhance the crystallization behavior. The recognition signal should be of short length and simple secondary structure. Thus, it can be easily introduced at different positions and without adversely affecting the biochemical activity of the molecule-of-interest which is completely synthesized at once. The sequence-specific recognition guarantees the high binding affinity required for stable complexation.

In a preferred embodiment of the present invention for generating a crystal containing a RNA, a linker is used which molecule-of-interest-binding site is PUM-HD, PABP, or a fragment thereof.
Puf proteins are developmental regulators that control mRNA stability and translation by binding sequences in the 3' untranslated regions of their target mRNAs. The founding member of this protein family is ***D**rosophila **m**elanogaster* **Pum**ilio (DmPUM) which binds specifically two tandem sequence motifs, **N**ano **R**esponse Elements (NRE). Each NRE contains two conserved regions, box A and box B. All Puf polypeptides contain a sequence-specific RNA-binding domain comprising eight sequence repeats and N- and C-terminal flanking regions, known as the Pumilio homology domain (PUM-HD). Minimal sequences of RNA were determined that could form stoichiometric complexes with PUM-HD and crystals. In the structure of the PUM-HD, RNA binds the concave surface of the molecule, and one base interacts with each repeat. This modular mode of RNA binding suggests that the polypeptide sequence specifically can be altered by mutating key residues within a single helical repeating unit (Wang *et al*. (2002) *Cell* 110, 501-512). The simple repeated motif of PUM-HD and its recognition mode predestine this polypeptide as molecule-of-interest-binding site of the linker. A general "code" to be discerned for base recognition may allow the engineering of the molecule-of-interest-binding site to exposed ssRNA sequences of the molecule-of-interest. Preferably, the sequence 5' AUU GUA CAU A 3' is used as recognition signal in the meaning of the present invention. It can be synthesized either at the 5'- or the 3'-terminus of the molecule-of-interest. Due to the molecular extension of PUM-HD which nearly corresponds to that of the recognition signal, no structural interference of the molecule-of-interest by PUM-HD is expected.

Poly(A)-binding protein (PABP) recognizes the 3' mRNA poly(A) tail and plays a critical role in eukaryotic translation initiation and mRNA stabilization/degradation. Truncated PABP containing RNA recognition motif (RRM) domains 3 and 4 retained binding to both AU-rich and oligo(A) RNA, whereas a truncated PABP containing RRM domains 1 and 2 was highly selective for oligo(A) RNA. The latter two RRMs form a continuous RNA-binding trough, lined by an antiparallel beta sheet backed by four alpha helices. The polyadenylate RNA adopts an extended conformation running the length of the molecular trough (Sladic et *al.* (2004) *Eur. J. Biochem.* 271, 450-457). The simple structure of the recognition signal and a dissociation constant in the nanomolar range for human PABP bound to AU-rich RNA and even sixfold stronger to oligo(A) RNA recommend this pair for usage in the present invention. Preferably, the sequence 5' (A)₁₁ 3' is used as recognition signal in the meaning of the present invention. It can be synthesized either at the 5'- or the 3'-terminus of the molecule-of-interest. Due to the molecular extension of PABP which nearly corresponds to that of the recognition signal, no structural interference of the molecule-of-interest by PABP is expected.

In another preferred embodiment of the invention for generating a crystal containing a RNA, monomeric streptavidin is used as crystallization carrier and a linker is used which carrier-binding site is a fM-Nano-tag (SEQ ID NO: 1 to 10) and which molecule-of-interest-binding site is Hsp15, (AAKK)₄, PUM-HD, PABP, or a fragment thereof.
The aforementioned combination is especially favorable for generating a crystal containing a RNA due to features of crystallization carrier and linker which has been characterized in the course of the present description.

According to the present invention, contacting the molecule-of-interest with the crystallization carrier by using the intermolecular linker can be carried out in different ways. In one embodiment of the invention, crystallization carrier and linker are contacted, thereby forming a complex, which is subsequently contacted with the molecule-of-interest, thereby forming the crystallizable complex. In another embodiment of the present invention, molecule-of-interest and linker are contacted, thereby forming a complex, which is subsequently contacted with the crystallization carrier, thereby forming the crystallizable complex. The preference of order depends on the specific components used. Probably, the first processing should be favored if the method is standardized and the sole remaining variable is the client-specific molecule-of-interest to be crystallized. However, it might also be practicable to incubate crystallization carrier, linker and molecule-of-interest simultaneously.

For contacting crystallization carrier, linker and molecule-of interest, binding buffer is used.
The binding buffer provides an environment which is necessary for strong association of binding partners. The environment may vary for association of crystallization carrier and linker as well as linker and molecule-of-interest requiring different types of buffer for each contacting step. Furthermore, the composition of binding buffer depends on the specific molecules to be contacted. Usually, it comprises ions like divalent cations for structure stabilization, oxidizing or reducing agents to produce the corresponding milieu, a buffer solution to set up the pH value etc.

The invention also relates to a crystal containing a molecule-of-interest which is generated by a method comprising:
a) contacting said molecule-of-interest with a crystallization carrier, the crystallization carrier being a well-crystallizing molecule structurally different from the molecule-of-interest and not being capable of homomultimerizing, using a heterologous intermolecular linker comprising a carrier-binding site and a molecule-of-interest-binding site, the linker being capable of interlinking crystallization carrier and molecule-of-interest or a recognition signal of the molecule-of-interest, thereby forming a crystallizable complex, whereby linker and molecule-of-interest are non-covalently interlinked, and crystallization carrier and molecule-of-interest are exclusively contacted by the linker; and
b) subjecting said complex to crystallization-inducing conditions which are derived from the crystallization conditions of the crystallization carrier and/or a complex consisting of crystallization carrier and linker, thereby generating a crystal containing the molecule-of-interest.

Mainly, the crystals are used for structure determination by X-ray structure analysis. Macromolecular crystals generated according to the teachings of the present invention also find important uses as catalysts, adsorbants, biosensors and chiral chromatographic media. They also may be employed in environmental applications, for example inbioremediation. In addition, crystals can be used as alternative way of drug delivery. Since the crystal is the most concentrated form of a biological macromolecule, such as a protein, it is of advantage if drugs require high doses at the delivery site. Since the rate of crystal dissolution depends on its morphology, size and the presence of excipients, crystalline proteins may also serve as a convenient slow release dosage form. Finally, due to the high stability of proteins in the crystalline form, crystals could be used to increase the drug shelf life.

The invention also relates to a method of determining the structure of a molecule-of-interest, wherein a crystal containing the molecule-of-interest is generated according to the prior teachings concerning the method of the present invention. The X-ray diffraction pattern of said crystal are recorded and by performing X-ray structure analysis of said crystal the structure of the molecule-of-interest is determined.
The carrier-driven crystallization of the present invention facilitates the determination of the three-dimensional structure of molecules that are either difficult to crystallize or do not crystallize at all. The inventive principle of using a crystallization carrier of known tertiary structure is appealing since the molecular replacement method using the known structure of the carrier can be applied more easily than for crystals of the molecule-of-interest alone. Respectively, isomorphous replacement is avoided which often represents the only applicable method for structure determination of a molecule-of-interest selected from biological macromolecules due to their size and complexity. Here, heavy atoms have to be introduced either during synthesis of the molecule-of-interest or by soaking into the crystal already grown. The first approach makes synthesis more difficult and interfere with the subsequent crystal growth sometimes. The second one does not inevitably lead to the incorporation of the heavy atom or could even destroy the crystal if it does. By applying the method of the present invention, the screening of suited heavy atoms for a specific molecule-of-interest or crystal containing it is avoided.

The invention also relates to a chimeric polypeptide linker for interlinking a crystallization carrier and a molecule-of-interest and for generating a crystal containing the molecule-of-interest, the polypeptide linker comprising:
a) a carrier-binding site being capable of non-covalently and specifically interlinking with a crystallization carrier, the crystallization carrier being a well-crystallizing molecule structurally different from the molecule-of-interest and not being capable of homomultimerizing;
b) a molecule-of-interest-binding site being capable of non-covalently interlinking with said molecule-of-interest; and optionally
c) a spacer peptide between carrier-binding site and molecule-of-interest-binding site.

Polypeptides are especially advantageously, because they are convenient, cheap and easily chemically synthesized, *in-vivo* expressed or *in-vitro* produced. There is a wide variety of peptide targets recognized by a potential molecule-of-interest, and there is the opportunity to select peptide ligands against any molecule useful as carrier-binding site and/or molecule-of-interest-binding site in the meaning of the invention. These peptide targets and/or ligands can be favorably combined to create binding sites for forming any ternary crystallizable complex. This merge of peptide sites of different origin and affinity in order to perform the task of generating a crystal, a function completely differing from each of the single peptide sites, is the actual essence of said polypeptide linker claimed by the present invention.
Eventually, an appropriate choice of crystallization carrier, cognate carrier-binding site and molecule-of-interest-binding site could be universally applied for a certain class of molecules-of-interest. Advantageously, at least one of the attached components is a protein, and more preferably at least the crystallization carrier is the protein, that the handling of the crystallizable complex is largely facilitated by the same molecule species. The non-covalent interlinkage of the crystallization carrier and the molecule-of-interest to the polypeptide linker ensures the proper folding of the aforementioned components into their native conformation.
Generally, single molecule linkers, being composed of covalently connected atoms, are highly stable and rigid. If applying short polypeptide linkers, preferably smaller than 20 amino acid residues, the rigid character of the peptide bond provides a conformational rigidity of the ternary crystallizable complex as well. At both binding sites, the cognate molecules bind and cover the polypeptide linker additionally providing stability and resulting in optimally ordered crystals. At best, the remaining uncovered linker structure is minimized, but without enabling cooperative binding of carrier and molecule-of-interest.
A larger polypeptide, preferably larger than 100 amino acid residues, may fulfill further functions in addition to interlinking. Depending on its crystallization behavior or the crystallization behavior of the complex of crystallization carrier and polypeptide linker, and the molecular weight of the linker, preferably being equivalent and, more preferably being greater than that of the molecule-of-interest, the polypeptide linker could act as second crystallization carrier. By providing novel crystal contacts to uniform molecular surfaces, any polypeptide linker regardless which length is also considered as crystallization enhancer. Large polypeptide linkers are preferably used at the molecule-of-interest-binding site.

In another embodiment of the present invention, the polypeptide linker is used for interlinking a crystallization carrier and a molecule-of-interest and for generating a crystal containing the molecule-of-interest.

In a preferred embodiment of the present invention for interlinking monomeric streptavidin as crystallization carrier and a RNA as molecule-of-interest, the polypeptide linker is a streptavidin-binding peptide covalently bound to a polypeptide selected from the group consisting of a RNA-backbone recognizing ligand, PUM-HD, PABP, or a fragment thereof.
This combination is especially favorable for interlinking carrier and molecule-of-interest and for generating a crystal containing a RNA due to features of crystallization carrier and linker which has been already characterized in the course of the present description.

In another preferred embodiment of the present invention, the polypeptide linker is used for interlinking monomeric streptavidin as crystallization carrier and a RNA as molecule-of-interest, the linker being a streptavidin-binding peptide covalently bound to a polypeptide selected from the group consisting of a RNA-backbone recognizing ligand, PUM-HD, PABP, or a fragment thereof.

In a preferred embodiment of the present invention, the carrier-binding site of the polypeptide linker for interlinking with monomeric streptavidin as crystallization carrier is a fM-Nano-tag (SEQ ID NO: 1 to 10).

The polypeptide linker and the matching crystallization carrier are selected such that when contacting the linker or a complex of linker and crystallization carrier with the molecule-of-interest, the crystallization carrier, the linker and the molecule-of-interest form a crystallizable complex of which a crystal containing the molecule-of-interest is generated when subjected to crystallization-inducing conditions.

All components of the chimeric polypeptide linker and the crystallizable complex have been characterized in detail in the course of the present description. The prior teaching of the present invention is considered as valid and without restrictions for all embodiments of the chimeric polypeptide linker. The embodiments of prior teaching of the present invention are also applicable to the chimeric polypeptide linker if expediently.

The invention also relates to a nucleic acid construct comprising a polynucleotide sequence which encodes a chimeric polypeptide linker for interlinking a crystallization carrier and a molecule-of-interest and for generating a crystal containing the molecule-of-interest, the polypeptide linker comprising:
a) a carrier-binding site being capable of non-covalently and specifically interlinking with a crystallization carrier, the crystallization carrier being a well-crystallizing molecule structurally different from the molecule-of-interest and not being capable of homomultimerizing;
b) a molecule-of-interest-binding site being capable of non-covalently interlinking with said molecule-of-interest; and optionally
c) a spacer peptide between carrier-binding site and molecule-of-interest-binding site.

The term "nucleic acid" refers to a natural or synthetic polymer of single- or doublestranded DNA or RNA alternatively including synthetic, non-natural or modified nucleotides which could be incorporated in DNA or RNA polymers. Each nucleotide consists of a sugar moiety, a phosphate moiety, and either a purine or pyrimidine residue.

In a preferred embodiment of the present invention, the nucleic acid construct is used for interlinking a crystallization carrier and a molecule-of-interest and for generating a crystal containing the molecule-of-interest.

In another preferred embodiment of the present invention for interlinking monomeric streptavidin as crystallization carrier and a RNA as molecule-of-interest, the nucleic acid construct encodes a streptavidin-binding peptide which can be covalently bound to a polypeptide selected from the group consisting of a RNA-backbone recognizing ligand, PUM-HD, PABP, or a fragment thereof.

In another preferred embodiment of the present invention, the nucleic acid construct is used for interlinking monomeric streptavidin as crystallization carrier and a RNA as molecule-of-interest, the nucleic acid construct encoding a streptavidin-binding peptide which is covalently bound to a polypeptide selected from the group consisting of a RNA-backbone recognizing ligand, PUM-HD, PABP, or a fragment thereof.

The encoded polypeptide linker and the matching crystallization carrier are selected such that when contacting the linker or a complex of linker and crystallization carrier with the molecule-of-interest, the crystallization carrier, the linker and the molecule-of-interest form a crystallizable complex of which a crystal containing the molecule-of-interest is generated when subjected to crystallization-inducing conditions.

All components of the nucleic acid construct comprising a polynucleotide sequence which encodes a chimeric polypeptide linker, and the crystallizable complex have been characterized in detail in the course of the present description. The prior teaching of the present invention is considered as valid and without restrictions for all embodiments of the nucleic acid construct. The embodiments of prior teaching of the present invention are also applicable to the nucleic acid construct if expediently.

The invention also relates to a kit for generating a crystal containing a molecule-of-interest comprising a crystallization carrier, the crystallization carrier being a well-crystallizing molecule structurally different from the molecule-of-interest and not being capable of homomultimerizing, a heterologous intermolecular linker comprising a carrier-binding site and a molecule-of interest-binding site, the linker being capable of interlinking crystallization carrier and molecule-of-interest or a recognition site thereof, thereby forming a crystallizable complex, whereby linker and molecule-of-interest are non-covalently interlinked, and crystallization carrier and molecule-of-interest are exclusively contacted by the linker, and crystallization solutions which are derived from the crystallization solutions for the crystallization of the crystallization carrier and/or crystallization solutions for the crystallization of a complex consisting of crystallization carrier and linker.
An information about the using of the kit should be kindly provided to guide the customer while generating a crystal containing a molecule-of-interest.

In an embodiment of the present invention, the kit comprises a binding buffer to interlink the crystallization carrier and the linker, or the crystallization carrier and a complex of linker and molecule-of-interest.

In another embodiment of the present invention, the kit comprises a binding buffer to interlink the molecule-of-interest and the linker, or the molecule-of-interest and a complex of linker and crystallization carrier.
This embodiment is only applicable if the linker universally interlinks a certain class of molecules-of-interest either directly or via a recognition signal thereof. Here, the binding is exclusively determined by features of the molecule-of-interest-binding site of the linker. Preferably, the molecule-of interest is a RNA and the cognate molecule-of-interest-binding site of the linker is a RNA-backbone recognizing ligand, PUM-HD, PABP, or a fragment thereof.

In another embodiment of the invention, the kit includes a complex consisting of crystallization carrier and linker.
Again, this embodiment is to be applied exclusively if the linker universally interlinks a certain class of molecules-of-interest either directly or via a recognition signal thereof. This pre-incubation of crystallization carrier and linker is regarded as customer-friendly. The user of the simple two-step kit starts by interlinking the molecule-of-interest with said complex ready-for-use, followed by subjecting the resulting crystallizable complex to crystallization solutions which are derived from the solutions for the crystallization of a complex consisting of crystallization carrier and linker. The crystallization solutions are favorably provided as sparse matrix screen.

The invention also relates to a kit for generating a crystal containing a molecule-of-interest comprising a crystallization carrier, the crystallization carrier being a well-crystallizing molecule structurally different from the molecule-of-interest and not being capable of homomultimerizing, a nucleic acid construct comprising a polynucleotide sequence which encodes a chimeric polypeptide linker comprising a carrier-binding site and a molecule-of interest-binding site, the linker being capable of interlinking crystallization carrier and molecule-of-interest or a recognition site thereof, thereby forming a crystallizable complex, whereby linker and molecule-of-interest are non-covalently interlinked, and crystallization carrier and molecule-of-interest are exclusively contacted by the linker, and crystallization solutions which are derived from the crystallization solutions for the crystallization of the crystallization carrier and/or crystallization solutions for the crystallization of a complex consisting of crystallization carrier and linker.

All components of the kit have been characterized in detail in the course of the present description. The prior teaching of the present invention is considered as valid and without restrictions for all embodiments of the kit. The embodiments of prior teaching of the present invention are also applicable to the kit if expediently.

It is to be understood that this invention is not limited to the particular methods, compositions and conditions described herein, as such matter may, of course, vary. It is also to be understood that the terminology used herein is for the purpose of describing particular embodiments only, and is not intended to limit the scope of the present invention.
As used herein, including the appended claims, singular forms of words such as "a," "an," and "the" include their corresponding plural referents unless the context clearly dictates otherwise. Thus, e.g., reference to "a crystal" includes one or more different crystals, reference to "a crystallization solution" includes one or more of such solutions, and reference to "a method" includes reference to equivalent steps and methods known to a person of ordinary skill in the art, and so forth.

### Example

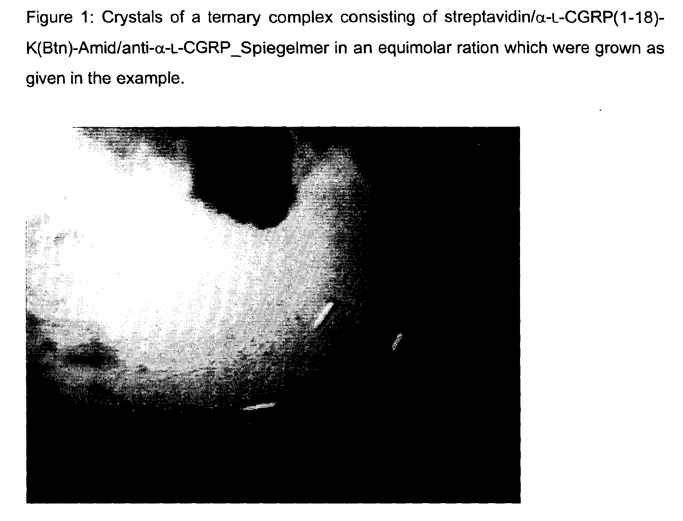

The following example is provided by way of illustration and not by way of limitation. Within the example, standard reagents and buffers that are free from contaminating activities (whenever practical) are used. It is preferred to exercise care to avoid ribonucleases.
The molecule-of-interest to be crystallized was the anti_α-L-CGRP_Spiegelmer (Vater *et al.* (2003) *NAR* 31, e130) which binds the target α-L-**c**alcitonin **g**ene-**r**elated peptide (α-L-CGRP) with nanomolar affinity. This neuropeptide is a potent endogenous vasodilator thought to be implicated in the genesis of migraine attacks. Intercepting α-L-CGRP by means of the Spiegelmer could treat migraine. However, insights into the structure/function relationship are still missing since several thousands of crystallization trials failed to generate crystals suited for recording a X-ray diffraction pattern.
In the below-mentioned example, the carrier-driven crystallization of the Spiegelmer as molecule-of-interest was tried and monomeric streptavidin was chosen as crystallization carrier. The linker was designed to contain a carrier-binding site to said streptavidin and a molecule-of-interest-binding site to the Spiegelmer. The latter site was represented by the Spiegelmer target peptide α-L-CGRP. Since the Spiegelmer recognizes the N-terminus of the target α-L-CGRP, the carrier-binding site was favorable added to the C-terminus. A lysine residue harboring a biotin moiety at the ε-amino group was covalently bound to a truncated target containing the amino acids 1 to 18. Since the residues 14 to 18 are not involved in Spiegelmer binding, they are a spacer peptide as defined according to the invention.
The following linker peptide derived from rat α-L-CGRP was synthesized using Fmoc chemistry: SCNTATCVTHRLAGLLSR-K(Btn)-Amid. Lyophilized peptide was solved in 10 mM sodium cacodylate (pH 5.5) to prevent the oxidation of cysteine residues in an alkaline milieu.
The anti_α-L-CGRP_Spiegelmer sequence 5' GGA CUG AUG GCG CGG UCC UAU UAC GCC GAA AGG GAG AGG GGA 3' was synthesized using standard phosphoramidite chemistry and then purified (Vater *et al*. (2003) *NAR* 31, e130). A monodisperse solution containing the Spiegelmer was generated by considering sample concentration (0.3 mM), folding temperature gradient (94°C for 5 min, 94°C to 37°C within 20 min) and salt conditions (10 mM HEPES (pH 7.4), 5 mM NaCl, 4 mM KCl, 5 mM MgCl₂, 1 mM CaCl₂). The monodisperse size distribution was confirmed by dynamic light-scattering experiments.
Recombinant, monomeric streptavidin was incubated with the 2.5-fold excess of linker peptide for 30 minutes at 37°C in binding buffer A (10 mM HEPES (pH 7.4), 5 mM NaCl, 4 mM KCI, 1 mM MgCl₂, 1 mM CaCl₂, 1 mM DTT). Unbound peptide was separated from the complex of carrier and linker by means of centrifugal filters YM-10 (Amicon) with 10 kDa molecular weight cut-off using washing buffer (10 mM HEPES (pH 7.4), 150 mM NaCl, 4 mM KCI, 1 mM MgCl₂, 1 mM CaCl₂, 1 mM DTT). The complex concentration was determined by UV measurement and Lambert-Beer equation. The stoichiometric binding of the linker to streptavidin could be confirmed by co-crystallization and X-ray structure analysis. The complex was subsequently incubated with an equimolar amount of Spiegelmer for 60 minutes at 37°C in binding buffer B (10 mM HEPES (pH 7.4), 150 mM NaCl, 4 mM KCl, 1 mM MgCl₂, 1 mM CaCl₂). Unbound Spiegelmer was not separated to ensure the saturation of the molecule-of-interest binding site. A buffer exchange by means of centrifugal filters YM-10 (Amicon) with 10 kDa molecular weight cut-off was optional. The complex concentration was determined by UV measurement and Lambert-Beer equation. The stoichiometric binding of the components within the crystallizable complex could be analyzed by electrophoretic mobility shift assay. The initial crystallization conditions were derived from that for tetrameric streptavidin (Schmidt *et al.* 1996 *J*. *Mol. Biol.* 255, 753-766) and comprise the variation of precipitant, pH (via the buffer), salt and concentrations thereof in the crystallization solutions (Table 1). 1 µl of crystallizable complex in different concentrations and 1 µl of crystallization solution were mixed and equilibrated against 800 µl of crystallization solution at several temperatures between 4 and 40°C. Thin, rod-shaped crystals (0.01 x 0.01 x 0.06 mm) were grown at 40°C in the well B2 (0.2 mM complex, 100 mM magnesium acetate, 50 mM sodium cacodylate (pH 6.5), 10 % w/v PEG 8000) within a couple of days (Figure 1) and cryo-protected by 160 mM magnesium acetate, 80 mM sodium cacodylate (pH 6.5), 16 % w/v PEG 8000 and 20 % v/v glycerol, for recording X-ray diffraction pattern under cryogenic conditions.

### Sequence Listing

SEQ ID NO: 1 Met Asp Val Glu Ala Trp

SEQ ID NO: 2 Met Asp Val Glu Ala

SEQ ID NO: 3 Met Asp Val Glu

SEQ ID NO: 4 Met Asp Val Xaa Xaa Trp

SEQ ID NO: 5 Met Asp Val Xaa Ala Trp

SEQ ID NO: 6 Met Asp Val Xaa Ala Trp Leu

SEQ ID NO: 7 Met Asp Val Xaa Ala Trp Leu Xaa

SEQ ID NO: 8 Met Asp Val Xaa Ala Trp Leu Xaa Xaa

SEQ ID NO: 9 Met Asp Val Xaa Ala Trp Leu Xaa Xaa Arg

SEQ ID NO: 10 Met Asp Val Xaa Ala Trp Leu Xaa Xaa Arg Val Pro Leu Val Glu Thr

### Sequence Listing Free Text

The sequences concern an optimized streptavidin-binding peptide with nanomolar affinity revealed by structural analysis. The methionine residue in position 1 of each sequence is modified by formylation. Xaa represents any amino acid.
From position 2 to 6, the present SEQ ID NO: 1 is identical to WO 03/074546 SEQ ID NO: 1.
From position 2 to 5, the present SEQ ID NO: 2 is identical to WO 03/074546 SEQ ID NO: 2.
From position 2 to 4, the present SEQ ID NO: 3 is identical to WO 03/074546 SEQ ID NO: 4.
From position 2 to 4 and in position 6, the present SEQ ID NO: 4 is identical to WO 03/074546 SEQ ID NO: 7.
From position 2 to 6 , the present SEQ ID NO: 5 is identical to WO 03/074546 SEQ ID NO: 7.
From position 2 to 7, the present SEQ ID NO: 6 is identical to WO 03/074546 SEQ ID NO: 8.
From position 2 to 8, the present SEQ ID NO: 7 is identical to WO 03/074546 SEQ ID NO: 9.
From position 2 to 9, the present SEQ ID NO: 8 is identical to WO 03/074546 SEQ ID NO: 10.
From position 2 to 10, the present SEQ ID NO: 9 is identical to WO 03/074546 SEQ ID NO: 11.
From position 2 to 16, the present SEQ ID NO: 10 is identical to WO 03/074546 SEQ ID NO: 12.

**Table 1: Crystallization solutions for subjecting a crystallizable complex containing monomeric streptavidin as crystallization carrier to crystallization-inducing conditions. The original crystallization solution (D2) for the crystallization of the crystallization carrier is bold.**

| **Well** | **Salt** | **Buffer** | **pH** | **Precipitant** |
|---|---|---|---|---|
| A1 | 0.1 M Cadmium Chloride | 0.1 M Sodium Acetate | *4.6* | 30 % w/v PEG 400 |
| B1 | 0.2 M Ammonium Acetate | 0.1 M tri-Sodium Citrate | *5.6* | 30 % w/v PEG 4000 |
| C1 | none | 0.1 M Sodium Acetate | *6.0* | 1.2 M Ammonium Sulfate |
| D1 | none | 0.1 M Sodium Acetate | *6.0* | 1.4 M Ammonium Sulfate |
| E1 | 0.2 M Potassium Bromide | 0.1 M Sodium Cacodylate | *6.5* | 10 % w/v PEG 1000, 10 % w/v PEG 8000 |
| F1 | 0.2 M Potassium Bromide | 0.1 M Sodium Cacodylate | *6.5* | 25 % w/v PEG 2000 MME |
| A2 | 0.08 M Magnesium Acetate | 0.05 M Sodium Cacodylate | *6.5* | 30 % w/v PEG 4000 |
| B2 | 0.2 M Magnesium Acetate | 0.1 M Sodium Cacodylate | *6.5* | 20 % w/v PEG 8000 |
| C2 | 0.2 M Sodium Acetate | 0.1 M Sodium Cacodylate | *6.5* | 30 % w/v PEG 8000 |
| D2 | none | 0.1 M Sodium Phosphate | 7.0 | 0.8 M Ammonium Sulfate |
| E2 | none | 0.1 M Sodium Phosphate | *7.0* | 1.0 M Ammonium Sulfate |
| F2 | none | 0.1 M Sodium Phosphate | *7.0* | 1.2 M Ammonium Sulfate |
| A3 | none | 0.1 M Sodium Phosphate | *7.0* | 1.4 M Ammonium Sulfate |
| B3 | 0.005 M Magnesium Chloride | 0.05 M HEPES - Na | 7.0 | 25 % v/v PEG 550 MME |
| C3 | none | 0.1 M HEPES - Na | *7.5* | 25 % w/v PEG 1000 |
| D3 | 0.3 M Sodium Acetate | 0.1 M Tris | *7.5* | 25 % w/v PEG 2000 MME |
| E3 | 0.2 M Lithium Sulfate | 0.1 M Tris | *7.5* | 25 % w/v PEG 2000 MME |
| F3 | none | 0.1 M HEPES - Na | *7.5* | 20 % w/v PEG 4000, 10% v/v iso-Propano |
| A4 | none | 0.1 M Sodium Phosphate | *8.0* | 1.0 M Ammonium Sulfate |
| B4 | none | 0.1 M Sodium Phosphate | *8.0* | 1.2 M Ammonium Sulfate |
| C4 | none | 0.1 M Sodium Phosphate | *8.0* | 1.4 M Ammonium Sulfate |
| D4 | none | 0.1 M Tris-HCl | *8.5* | 2.0 M Ammonium Sulfate |
| E4 | 0.2 M Ammonium Chloride, 0.01 M Calcium Chloride | 0.05 M Tris-HCl | *8.5* | 30 % w/v PEG 4000 |
| F4 | 0.2 M Sodium Acetate | 0.1 M Tris-HCl | *8.5* | 30 % w/v PEG 4000 |

## Claims

1. A method of generating a crystal containing a molecule-of-interest comprising:
a) contacting said molecule-of-interest with a crystallization carrier, the crystallization carrier being a well-crystallizing molecule structurally different from the molecule-of-interest and not being capable of homomultimerizing, using a heterologous intermolecular linker comprising a carrier-binding site and a molecule-of-interest-binding site, the linker being capable of interlinking crystallization carrier and molecule-of-interest or a recognition signal thereof, thereby forming a crystallizable complex, whereby linker and molecule-of-interest are non-covalently interlinked, and crystallization carrier and molecule-of-interest are exclusively contacted by the linker; and
b) subjecting said complex to crystallization-inducing conditions which are derived from the crystallization conditions of the crystallization carrier and/or a complex consisting of crystallization carrier and linker, thereby generating a crystal containing the molecule-of-interest.

2. The method according to claim 1, wherein a protein or a nucleic acid is used as molecule-of-interest.

3. The method according to claim 1, wherein the crystallization carrier is a protein, preferably calmodulin, S-protein or monomeric streptavidin.

4. The method according to claim 3, wherein mutated streptavidin is used.

5. The method according to claim 1, wherein a linker is used which contains a spacer molecule between carrier-binding site and molecule-of-interest-binding site.

6. The method according to claim 1, wherein a linker is used which carrier-binding site is a peptide.

7. The method according to claims 1 and 6, wherein monomeric streptavidin is used as crystallization carrier and a linker is used which carrier-binding site is a streptavidin-binding peptide.

8. The method according to claim 7, wherein a linker is used which carrier-binding site comprises the amino acid sequences of SEQ ID NOs: 1 to 10.

9. The method according to claim 1, wherein a linker is used which is a polypeptide or which molecule-of-interest-binding site is a polypeptide or a peptide.

10. The method according to claim 1 and 9 for generating a crystal containing a RNA, wherein a linker is used which molecule-of-interest-binding site is a RNA-backbone recognizing ligand or a fragment thereof.

11. The method according to claims 1 and 9 for generating a crystal containing a RNA, wherein a linker is used which molecule-of-interest-binding site is PUM-HD, PABP, or a fragment thereof.

12. The method according to claims 1 and 9 for generating a crystal containing a RNA, wherein monomeric streptavidin is used as crystallization carrier and a linker is used which carrier-binding site is a fM-Nano-tag comprising one of sequences of SEQ ID NOs: 1 to 10, and which molecule-of-interest-binding site is Hsp15, (AAKK)₄, PUM-HD, PABP, or a fragment thereof.

13. A crystal generated by the method of claim 1.

14. A method of determining the structure of a molecule-of-interest, wherein a crystal containing the molecule-of-interest is generated according to claims 1 to 12, the X-ray diffraction pattern of said crystal are recorded and by performing X-ray structure analysis of said crystal the structure of the molecule-of-interest is determined.

15. A chimeric polypeptide linker for interlinking a crystallization carrier and a molecule-of-interest and for generating a crystal containing the molecule-of-interest, the polypeptide linker comprising:
a) a carrier-binding site being capable of non-covalently and specifically interlinking with a crystallization carrier, the crystallization carrier being a well-crystallizing molecule structurally different from the molecule-of-interest and not being capable of homomultimerizing;
b) a molecule-of-interest-binding site being capable of non-covalently interlinking with said molecule-of-interest; and optionally
c) a spacer peptide between carrier-binding site and molecule-of-interest-binding site.

16. The polypeptide linker according to claim 15 for interlinking monomeric streptavidin as crystallization carrier and a RNA as molecule-of-interest, being a streptavidin-binding peptide covalently bound to a polypeptide selected from the group consisting of a RNA-backbone recognizing ligand, PUM-HD, PABP, or a fragment thereof.

17. Use of the polypeptide linker according to claim 16 for interlinking monomeric streptavidin as crystallization carrier and a RNA as molecule-of-interest, being a streptavidin-binding peptide covalently bound to a polypeptide selected from the group consisting of a RNA-backbone recognizing ligand, PUM-HD, PABP, or a fragment thereof.

18. A nucleic acid construct comprising a polynucleotide sequence which encodes a chimeric polypeptide linker for interlinking a crystallization carrier and a molecule-of-interest and for generating a crystal containing the molecule-of-interest, the polypeptide linker comprising:
a) a carrier-binding site being capable of non-covalently and specifically interlinking with a crystallization carrier, the crystallization carrier being a well-crystallizing molecule structurally different from the molecule-of-interest and not being capable of homomultimerizing;
b) a molecule-of-interest-binding site being capable of non-covalently interlinking with said molecule-of-interest; and optionally
c) a spacer peptide between carrier-binding site and molecule-of-interest-binding site.

19. The nucleic acid construct according to claim 18 for interlinking monomeric streptavidin as crystallization carrier and a RNA as molecule-of-interest, encoding a streptavidin-binding peptide which can be covalently bound to a polypeptide selected from the group consisting of a RNA-backbone recognizing ligand, PUM-HD, PABP, or a fragment thereof.

20. Use of the nucleic acid construct according to claim 19 for interlinking monomeric streptavidin as crystallization carrier and a RNA as molecule-of-interest, encoding a streptavidin-binding peptide which can be covalently bound to a polypeptide selected from the group consisting of a RNA-backbone recognizing ligand, PUM-HD, PABP, or a fragment thereof.

21. A kit for generating a crystal containing a molecule-of-interest comprising a crystallization carrier, the crystallization carrier being a well-crystallizing molecule structurally different from the molecule-of-interest and not being capable of homomultimerizing, a heterologous intermolecular linker comprising a carrier-binding site and a molecule-of interest-binding site, the linker being capable of interlinking crystallization carrier and molecule-of-interest or a recognition site thereof, thereby forming a crystallizable complex, whereby linker and molecule-of-interest are non-covalently interlinked, and crystallization carrier and molecule-of-interest are exclusively contacted by the linker, and crystallization solutions which are derived from the crystallization solutions of the crystallization carrier and/or a complex consisting of crystallization carrier and linker.

22. The kit according to claim 21, comprising a binding buffer to interlink the crystallization carrier and the linker, or the crystallization carrier and a complex of linker and molecule-of-interest.

23. The kit according to claim 21, wherein a complex consisting of crystallization carrier and linker is included.

24. A kit for generating a crystal containing a molecule-of-interest comprising a crystallization carrier, the crystallization carrier being a well-crystallizing molecule structurally different from the molecule-of-interest and not being capable of homomultimerizing, a nucleic acid construct comprising a polynucleotide sequence which encodes a chimeric polypeptide linker comprising a carrier-binding site and a molecule-of interest-binding site, the linker being capable of interlinking crystallization carrier and molecule-of-interest or a recognition site thereof, thereby forming a crystallizable complex, whereby linker and molecule-of-interest are non-covalently interlinked, and crystallization carrier and molecule-of-interest are exclusively contacted by the linker, and crystallization solutions which are derived from the crystallization solutions of the crystallization carrier and/or a complex consisting of crystallization carrier and linker.

25. Streptavidin-binding peptide comprising the amino acid sequences of SEQ ID NOs: 1 to 10.

26. Use of a streptavidin-binding peptide according to claim 25
- as carrier-binding site of a heterologous intermolecular linker in the crystallization method according to claim 1; or
- for purification of a protein produced in a system for protein biosynthesis; or
- for labeling of a protein; or
- for immobilization of proteins to protein lattices containing streptavidin, thereby generating biochips.


SEQUENCE LISTING

<110> Freie Universitaet Berlin

<120> A method of generating a crystal of a
      molecule-of-interest and a kit therefor

<130> P293104EP

<140>
<141>

<160> 10

<170> PatentIn Ver. 2.1

<210> 1
<211> 6
<212> PRT
<213> Artificial Sequence

<220>
<223> Description of Artificial Sequence: Optimized
      streptavidin-binding peptide with nanomolar
      affinity revealed by structural analysis.

<220>
<221> MOD_RES
<222> (1)
<223> FORMYLATION

<220>
<221> SIMILAR
<222> (2)..(6)
<223> identical to WO 03/074546 SEQ ID NO: 1

<400> 1
Met Asp Val Glu Ala Trp
  1               5


<210> 2
<211> 5
<212> PRT
<213> Artificial Sequence

<220>
<223> Description of Artificial Sequence: Optimized

streptavidin-binding peptide with nanomolar
      affinity revealed by structural analysis.

<220>
<221> MOD_RES
<222> (1)
<223> FORMYLATION

<220>
<221> SIMILAR
<222> (2)..(5)
<223> identical to WO 03/074546 SEQ ID NO: 2

<400> 2
Met Asp Val Glu Ala
  1               5


<210> 3
<211> 4
<212> PRT
<213> Artificial Sequence

<220>
<223> Description of Artificial Sequence: Optimized
      streptavidin-binding peptide with nanomolar
      affinity revealed by structural analysis.

<220>
<221> MOD_RES
<222> (1)
<223> FORMYLATION

<220>
<221> SIMILAR
<222> (2)..(4)
<223> identical to WO 03/074546 SEQ ID NO: 4

<400> 3
Met Asp Val Glu
  1


<210> 4
<211> 6
<212> PRT
<213> Artificial Sequence

<220>
<223> Description of Artificial Sequence: Optimized
      streptavidin-binding peptide with nanomolar
      affinity revealed by structural analysis.

<220>
<221> MOD_RES
<222> (1)
<223> FORMYLATION

<220>
<221> SIMILAR
<222> (2)..(4)
<223> identical to WO 03/074546 SEQ ID NO: 7

<220>
<221> SIMILAR
<222> (6)
<223> identical to WO 03/074546 SEQ ID NO: 7

<220>
<221> VARIANT
<222> (4)..(5)
<223> any amino acid

<400> 4
Met Asp Val Xaa Xaa Trp
  1               5

<210> 5
<211> 6
<212> PRT
<213> Artificial Sequence

<220>
<223> Description of Artificial Sequence: Optimized
      streptavidin-binding peptide with nanomolar
      affinity revealed by structural analysis.

<220>
<221> MOD_RES
<222> (1)
<223> FORMYLATION

<220>
<221> SIMILAR
<222> (2)..(6)

<223> identical to WO 03/074546 SEQ ID NO: 7

<220>
<221> VARIANT
<222> (4)
<223> any amino acid

<400> 5
Met Asp Val Xaa Ala Trp
1               5


<210> 6
<211> 7
<212> PRT
<213> Artificial Sequence

<220>
<223> Description of Artificial Sequence: Optimized
      streptavidin-binding peptide with nanomolar
      affinity revealed by structural analysis.

<220>
<221> MOD_RES
<222> (1)
<223> FORMYLATION

<220>
<221> SIMILAR
<222> (2)..(7)
<223> identical to WO 03/074546 SEQ ID NO: 8

<220>
<221> VARIANT
<222> (4)
<223> any amino acid

<400> 6
Met Asp Val Xaa Ala Trp Leu
1               5


<210> 7
<211> 8
<212> PRT
<213> Artificial Sequence

<220>

<223> Description of Artificial Sequence: Optimized
      streptavidin-binding peptide with nanomolar
      affinity revealed by structural analysis.

<220>
<221> MOD_RES
<222> (1)
<223> FORMYLATION

<220>
<221> SIMILAR
<222> (2)..(8)
<223> identical to WO 03/074546 SEQ ID NO: 9

<220>
<221> VARIANT
<222> (4)
<223> any amino acid

<220>
<221> VARIANT
<222> (8)
<223> any amino acid

<400> 7
Met Asp Val Xaa Ala Trp Leu Xaa
  1               5

<210> 8
<211> 9
<212> PRT
<213> Artificial Sequence

<220>
<223> Description of Artificial Sequence: Optimized
      streptavidin-binding peptide with nanomolar
      affinity revealed by structural analysis.

<220>
<221> MOD_RES
<222> (1)
<223> FORMYLATION

<220>
<221> SIMILAR
<222> (2)..(9)
<223> identical to WO 03/074546 SEQ ID NO: 10

<220>
<221> VARIANT
<222> (4)
<223> any amino acid

<220>
<221> VARIANT
<222> (8)..(9)
<223> any amino acid

<400> 8
Met Asp Val Xaa Ala Trp Leu Xaa Xaa
1               5

<210> 9
<211> 10
<212> PRT
<213> Artificial Sequence

<220>
<223> Description of Artificial Sequence: Optimized
      streptavidin-binding peptide with nanomolar
      affinity revealed by structural analysis.

<220>
<221> MOD_RES
<222> (1)
<223> FORMYLATION

<220>
<221> SIMILAR
<222> (2)..(10)
<223> identical to WO 03/074546 SEQ ID NO: 11

<220>
<221> VARIANT
<222> (4)
<223> any amino acid

<220>
<221> VARIANT
<222> (8)..(9)
<223> any amino acid

<400> 9
Met Asp Val Xaa Ala Trp Leu Xaa Xaa Arg

1                 5                   10

<210> 10
<211> 16
<212> PRT
<213> Artificial Sequence

<220>
<223> Description of Artificial Sequence: Optimized
      streptavidin-binding peptide with nanomolar
      affinity revealed by structural analysis.

<220>
<221> MOD_RES
<222> (1)
<223> FORMYLATION

<220>
<221> SIMILAR
<222> (2)..(16)
<223> identical to WO 03/074546 SEQ ID NO: 12

<220>
<221> VARIANT
<222> (4)
<223> any amino acid

<220>
<221> VARIANT
<222> (8)..(9)
<223> any amino acid

<400> 10
Met Asp Val Xaa Ala Trp Leu Xaa Xaa Arg Val Pro Leu Val Glu Thr
 1               5                   10                  15